(19)

Europäisches
Patentamt

European
Patent Office

Office européen
des brevets

(11) **EP 4 511 601 B1**

(12) **EUROPEAN PATENT SPECIFICATION**

(45) Date of publication and mention
of the grant of the patent:
**24.09.2025 Bulletin 2025/39**

(21) Application number: **23720096.9**

(22) Date of filing: **18.04.2023**

(51) International Patent Classification (IPC):
$F24F\ 1/00$ (2019.01)     $F28F\ 27/00$ (2006.01)
$G01N\ 27/22$ (2006.01)

(52) Cooperative Patent Classification (CPC):
**G01N 27/221; G01N 33/2841; F28F 27/00;**
**G01F 23/263**

(86) International application number:
**PCT/EP2023/059991**

(87) International publication number:
**WO 2023/203019 (26.10.2023 Gazette 2023/43)**

(54) **DEVICE AND METHOD FOR MEASURING TOTAL GAS CONTENT**

VORRICHTUNG UND VERFAHREN ZUR MESSUNG DES GESAMTGASGEHALTS

DISPOSITIF ET PROCÉDÉ DE MESURE DE TENEUR TOTALE EN GAZ

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**

(30) Priority: **19.04.2022  SE 2250468**
**19.04.2022  SE 2250469**

(43) Date of publication of application:
**26.02.2025  Bulletin 2025/09**

(73) Proprietor: **QTF Sweden AB**
**393 53 Kalmar (SE)**

(72) Inventor: **CARLSSON, Björn**
**392 43 KALMAR (SE)**

(74) Representative: **Noréns Patentbyrå AB**
**Box 10198**
**100 55 Stockholm (SE)**

(56) References cited:
**JP-A- S5 282 358     US-A1- 2015 160 147**
**US-B1- 6 272 906     US-B1- 6 318 332**

**Description**

Field of the invention

This invention relates to a system comprising a heating system or a cooling system and a device for measuring total gas content in a liquid.

Background

Energy transfer systems such as heat pumps and cooling systems use liquids for transfer of heat for example between a cold side and a hot side of a system. Examples of such liquids include water, brine, glycols and various oils. Such liquids contain a certain amount of gas dissolved therein, for various reasons. Gas may for example enter the system when filling liquid or due to leakage. Gas may also diffuse into the system, in particular when the system comprises tubes or pipes made of plastic. Moreover, some liquids form gases over time.

This poses a problem because gas in a system for liquids may disturb flow and pump pressure. For example, the pressure may decrease locally in a pump so that the gas forms bubbles which may cause cavitation. Gas may also cause corrosion in the system. Gas bubbles may decrease pump efficacy. Gas may also decrease heat transfer efficacy by blocking surface area of heat exchangers.

For this reason, such systems may have valves or other devices for degassing the liquid therein. However, it is often difficult to know when a system needs to be degassed. The rate of collection of gas in the system may vary over time. Hence, there is a need for a device for easily monitoring the amount of total gas in the system. Such a device can for example be used to know when the system needs to be degassed.

Various systems for analysis specific compounds in liquids, such as oxygen or carbon dioxide, are known. However, such devices do not determine the amount of total gas unless a specific analysis device is used for each and every compound that is expected to be in the system.

US 2015/0160147 A1 relates to methods and systems for characterizing void fractions in heat transfer systems for cooling and/or heating including determining a void fraction dependent parameter by measuring a capacitance over or in a channel wherein the multi-phase system occurs.

JPS 5282358 A discloses an apparatus for gas volume measurement. The apparatus comprises an analysis container with means for decreasing the pressure therein. A piston is moved to displace the mixture in the analysis container. First and second electrical poles are used to determine the presence of liquid in a pipe connected to the analysis container. When liquid is detected, the gas volume in the analysis container is determined by reading the piston position.

US 6,272,906 B1 refers to a phase separator intended to separate and to measure the volume of the various phases of a mixture of fluids.

US 6,318,332 B1 describes a method for monitoring adequate oil lubrication of an internal combustion engine.

Hence there is a need for a device for easily measuring the amount of total gas in a closed liquid system.

There is also a need for a device for measuring total gas content in body of water (not according to the present invention), such as the sea, a lake or a river. For example, environmental waste may release large amounts of gas such as hydrogen sulfide.

Summary of invention

The system of the present invention is defined in claim 1. Further, a method for determining total gas in the system of claim 1 is defined in claim 5.

This provides a simple and reliable means of measuring total gas content. Any type of gas can be detected.

Drawings

Fig. 1 is a schematic drawing of a system, where a device is seen from the side.
Fig. 2 is a schematic top view of a gas collecting space.
Fig. 3 is a schematic top view of a gas collecting space.
Fig. 4 is a schematic drawing of a gas colleting space seen from the side.
Fig. 5 is a schematic drawing of a gas colleting space seen from the side.
Fig. 6 is a flowchart that shows a method.
Fig. 7 is a schematic drawing showing various examples of a water analysis device. The water analysis device is not in accordance with the present invention.

Detailed description

"Total gas" may refer to any gas that is dissolved in the liquid such as for example oxygen, nitrogen, carbon dioxide or noble gasses. "Total gas" includes gases that are dissolved in the liquid and also gases that become liquids by forming chemical compounds at a high pressure but become gases at lower pressures. Examples of such gases are carbon dioxide, which to a large extent forms carbonic acid in water at high pressure but become gaseous carbon dioxide at lower pressure. Similar procedures occur with ammonia and hydrogen sulfide.

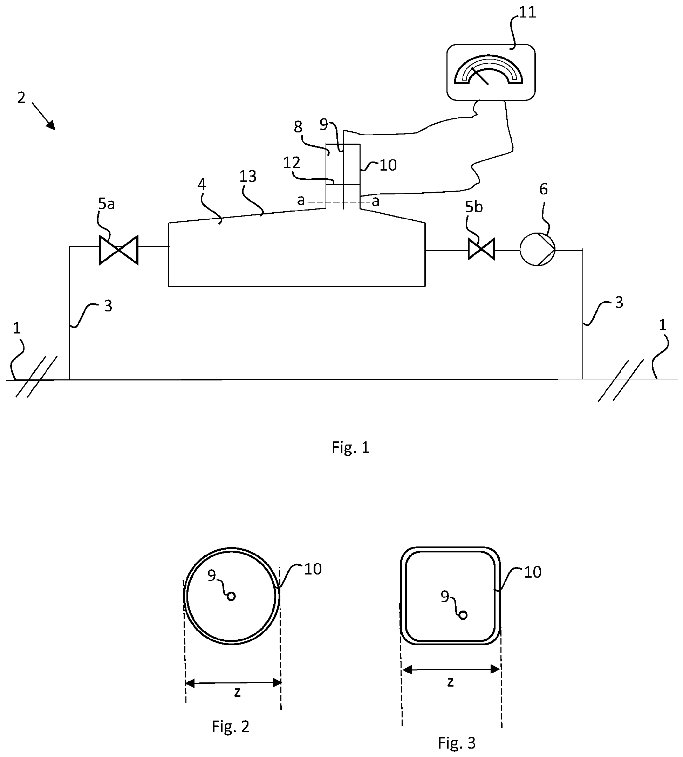

With reference to Fig. 1, the system 1 is a part of a heating system or a cooling system, such as a part of a heat exchanger or other device for transfer of heat. Examples of systems include heat pumps or other systems for extracting heat or cold from for example drilled energy wells, bodies of water, waste heat in industry or wastewater, district heating systems and district cooling systems and large-scale freezers such as freezers in supermarkets. System 1 contains a liquid which may be for example water, brine, glycol, alcohol or mixtures these, oil, or aqueous solutions of organic or non-organic salts. System 1 is closed such that liquid or gas cannot leave the system unless a valve or similar is opened. System 1 may comprise a number of different pipes or tanks, and may also comprise tanks or valves for controlling the flow of liquid. Typically, the liquid in system 1 is circulating, for example with the aid of one or more pumps.

Device 2 is connected to the system 1 with conduits 3. Device 2 comprises means for isolating a part of the liquid in the system 1 from the rest of the system 1. The means for isolating a part of the liquid is an analysis container 4 which is connected to system 1 but which can be disconnected from system 1, for example with the aid of valves 5a, 5b. The volume of the analysis container 4 is preferably known. The analysis container 4 is connected to means for decreasing the pressure in the analysis container 4 such as a pump 6. The means for decreasing the pressure in the analysis container may alternatively be means for increasing the volume of the analysis container 4. For example, a side wall of the analysis container 4 may be flexible such that it can alter between an inward bulging state and an outward bulging state. In yet a different embodiment the analysis container 4 is a cylinder with a piston that can move to alter the pressure inside.

Hence a sample of the liquid in system 1 can be isolated from the rest of the system 1 using the valves 5a, 5b. The sample is then subjected to low pressure with the aid of pump 6. A suitable final pressure in the analysis container 4 may be 0.5-0.6 bar ATA. Valve 5b may be a one-way valve.

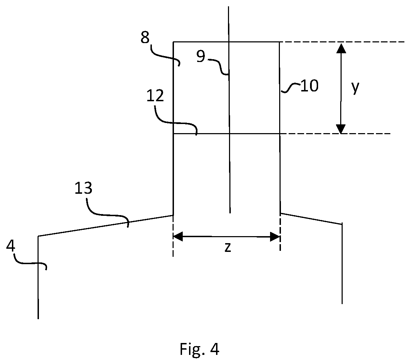

With reference to Figs 1 to 5, the top of the analysis container 4 is in fluid connection with a gas collecting space 8 arranged above the analysis container 4. The gas collecting space 8 is elongated and has a main axis that is non-horizontal, preferably vertical (as seen in the figures). The gas collecting space 8 has the same width (z) along its main axis (Fig. 4). The gas collecting space 8 preferably has the shape of a cylinder. The gas collection space 8 may extend non-horizontally, preferably vertically, from the analysis container 4 as seen in Figs 1 and 4.

Any fluid connection between the analysis container 4 and the gas collecting space 8 should preferably be arranged so that bubbles are not trapped, but that all gas bubbles are collected in the gas collecting space 8. The upper part 13 of the analysis container 4 may be funnel shaped as seen in Fig. 1 in order to avoid trapping of gas bubbles in the upper part of the analysis container 4 but to funnel gas to the gas collecting space 8.

Decreasing the pressure in the analysis container 4 will make gas in the liquid collect in the gas collecting space 8. A horizontal interface 12 between the liquid and the gas will form in the gas collecting space 8.

The analysis container 4 may have any suitable volume for example from 0.1 to 10 liters but other volumes are possible depending on the gas measured and other conditions. The volume of the gas collecting space 8 is selected depending on which gases can be expected to be present in system 1, which depends on the liquid in the system 1 and other factors. A suitable volume of the gas collecting space 8 may be at least 5 % of the volume of the analysis container 4. When the liquid is water-based and the system 1 is expected to contain carbon dioxide, the volume of gas collecting space 8 may have to be much larger, such as several times the volume of the analysis container 4. The same considerations may have to be made when other gases that can be present in large amounts in the liquid in question. A gas collecting space 8 with a large volume will result in a device 2 with a lower sensitivity but a larger detection range, whereas a smaller volume of the gas collecting space 8 will result in higher sensitivity but smaller detection range.

The gas collecting space 8 forms a capacitor where a first electrical pole 9 is an elongated member arranged in the gas collecting space 8 such that it extends through the interface 12 between the liquid and the gas, and a second pole 10 on the inner surface of the gas collecting space 8. The first pole 9 is preferably elongated such as for example a rod or a wire. The capacitance between the first pole 9 and second poles 10 is used to determine the amount of gas in the gas collecting space 8. There is an open space between the first pole 9 and the second pole 10 which is filled by liquid or gas. The first pole 9 may be arranged in the middle of the gas collecting space 8. The first pole 9 is parallel to the main axis of the gas collecting space 8. The first pole 9 may have a length such that it does not extend below the gas collecting space 8 as seen in the figures.

The fist pole 9 is preferably made from a conducting material which is insulated. The first pole 9 may be a metal wire or a metal rod, such as a copper wire. The second pole 10 is preferably made of a conducting metal such as stainless steel.

As mentioned above, the gas collecting space 8 has a non-horizontally elongated shape. The gas collecting space 8 has an even width. This has the advantage of providing a linear relationship between the capacitance and the gas volume as described below.

Fig. 2 shows the section of the gas collection space 8 seen along the line a-a in Fig. 1. The gas collecting space 8 may have any profile such as square or oval, but is preferably circular as in Fig. 2. The interior area A of the cross-section a-a of the gas collecting space 8 may be known.

Fig. 3 shows a different example of how the section a-a of the gas collection interface can be arranged. Here the section a-a is shaped like a square and the first pole 9 is not arranged in the middle of the gas collecting space 8. Arranging the first pole 9 closer to one wall of the second pole 10 will decrease the capacitance compared to placing the first pole 9 in the middle like in Fig. 2.

A non- limiting example of how the capacitance between the first pole 9 and the second pole 10 will now be described. However, the skilled person understands that different approaches may be employed to use the capacitance between the first pole 9 and the second pole 10 to determine the amount of gas in the gas collecting space.

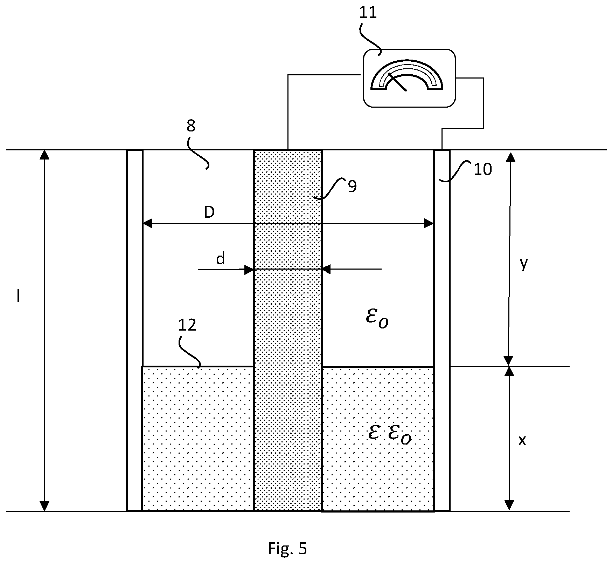

The capacitance C for a cylindrical capacitor as shown in Fig. 5, which is essentially the same arrangement as in Fig. 2, can be determined as

$$C = \frac{2\pi \, \varepsilon \, \varepsilon_o \, l}{\ln\frac{D}{d}} \qquad\qquad (1)$$

where

  $\varepsilon$= the relative dielectric constant for the material
  $\varepsilon_o$ = the dielectric constant for air
  l = length of the capacitor
  D= inner diameter of the capacitor
  d=diameter of the first pole

In general, the dielectric constant is much lower for gases than for liquids and the dielectric constant for air can be used in most cases.

The capacitance of the capacitor of Fig. 5 can be determined as

$$C = \frac{2\pi \, \varepsilon \, \varepsilon_o \, x}{\ln\frac{D}{d}} + \frac{2\pi \, \varepsilon \, (l-x)}{\ln\frac{D}{d}} \qquad\qquad (2)$$

Hence, there will be a linear relationship between the amount of gas in the gas collection space 8 and the capacitance. This relationship can be used to determine x (x being the height of the liquid along the first pole 9 in the gas collecting space 8) and then y (y=l-x) in Fig 5. By measuring the capacitance between the first 9 and second poles 10 with the use of a capacitance meter 11, the height y of the gas pillar in the gas collecting space 8 can be determined. The height y of the gas pillar will be representative of the gas content in the liquid. The diameter D or the area A of the section of the gas collecting space 8 is preferably known. Since the volume of the analysis container 4 is preferably known, the total gas content in the liquid in the system 1 can be determined.

The above calculation is suitable for a cylindrical gas collecting space 8. However, any suitable method may be used to relate the capacitance to the volume of gas. For example, manual /or brute force type calibrations may be used. For example, a threshold capacitance may be used such that a signal is provided when the capacitance is above the threshold capacitance. Such a signal is indicative that the total gas concentration in the liquid is over a threshold.

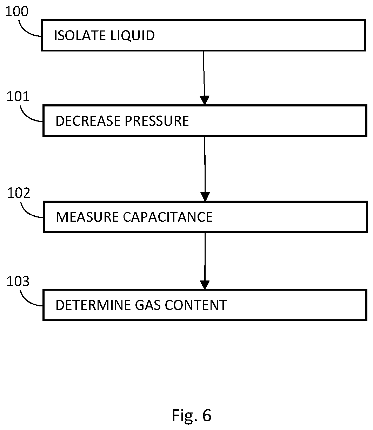

With reference to Fig. 6 a method may comprise the steps of 100 isolating a portion of the liquid from the system 1 in the analysis container 4, for example by closing valves 5a and 5b. In step 101 the pressure in the analysis container 4 is decreased. This will cause gas to form in the gas collection space 8. In step 102 the capacitance is measured. In step 103 the capacitance is converted to data representing gas content of the liquid.

The upper end of the gas collecting space may have a degassing valve 14 for releasing the collected gas after measurement. A new sample may then be analyzed.

The capacitance signal detected by the capacitance meter 11 may be treated or processed in any suitable manner. The device 2 may have suitable control circuitry powered by an energy source such as electric power provided from an outlet or a battery. The capacitance signal may for example be digitalized, for example using a A/D converter. However, an analogue signal may be used. The capacitance signal may be converted to a value or data representing gas content, for example using the linear relationship described above. Any suitable combination of hardware and software may be used to treat or process the capacitance signal. The device may for example comprise a memory, a processor and a bus. Any suitable programming language can be used. The device 2 may have an output means for providing data to a user, such as a display for providing information to a user or a communications interface, such as a network interface for providing data to a second device. The device may be arranged to provide a signal corresponding to the total gas content. A capacitance threshold may be used to trigger an alarm, indicating too much gas in the system 1.

Water analysis device (not according to the present invention)

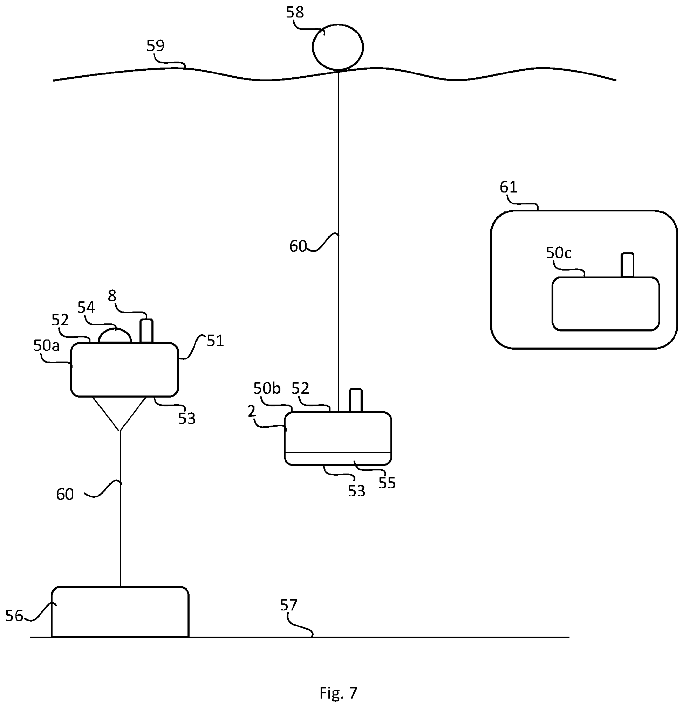

With reference to Fig. 7, there is also provided a water analysis device 50 for measuring total gas in a body of water which works in essentially the same manner as the device described above. The water analysis device is arranged to be submerged in a body of water such as the sea, a lake or a river. The device provides a simple and reliable device for measuring total gas content in a body of water.

The water analysis device 50 has means for isolating a sample from the body of water such as analysis container 4 and a valve for letting in water into the analysis container. The degassing valve 14 may be opened to allow a sample of water to enter the analysis container 4 by allowing gas to escape from the analysis container. The device 50 may have a pump for exchanging the water in the analysis container 4.

Fig. 7 shows an example where a water analysis device 50abc is used for measuring total gas content in a body of water. The water analysis device 50 may be comprised in a housing 51 where the gas collecting space 8 is attached on top of the housing 51. The gas collecting space 8 may alternatively be comprised inside the housing 51. Water analysis device 50a is buoyant and is anchored with an anchor 56 that rests on the seabed 57. Water analysis device 50b is heavier than water and is suspended from a buoy 58 that floats on the water surface 59.

The water analysis device 50 may have suitable attachment means for suspending or anchoring the device in a body of water, such as a noose or a shackle for attaching an anchor 56 or a buoy 58 via wire or rope 60.

The water analysis device 50 has an upper end 52 and a lower end 53 and the device 50 is preferably arranged to be arranged to maintain a level orientation in the water. For example, the water analysis device 50 may have a flotation device 54 in the top end, or a weight 55 in the bottom end, or both. The water analysis device 50 is preferably balanced such that filling the gas collecting space 8 with gas does not change the orientation of the device 50 in a manner that affects the readout. Looking at the suspended water analysis device 50b it may for example be suitable to place the gas collecting space 8 as close to the suspension wire 60 as possible or order to maintain the device 50 level when the gas colleting space 8 is filled with gas.

The device 50 may provide a signal corresponding to a gas amount via a cable to a wireless communication device which may be arranged on buoy 58.

Device 50c is an example where the water analysis device 50c is aboard a remotely operated vehicle or underwater drone 61. Such a vehicle 61 may provide a data storage data facility with a port for extracting stored data after a mission.

The water analysis device 50 is preferably watertight so that it can be submerged to a suitable depth in the body of water. The water analysis device 50 may be arranged to withstand at least 0.3 bars of pressure which is the pressure at a depth of 3 meters. The water analysis device may alternatively be arranged to withstand a pressure of at least 9.78 bar which is the pressure at a depth of 100 m. Hence the housing 50 may be arranged to protect various parts of the water analysis device 50 such as control circuitry, battery, the analysis container 4 and the gas collecting space 8.

By placing the device 50 in a body of water, for example at a suitable depth, the total gas at the depth may be measured in a convenient manner.

A method for determining total gas in sample from a body of water using a water analysis device the method may comprise the steps placing the device in the body of water, such that the upper end is directed upwards towards the surface and the lower end is directed towards the bed of the body of water, isolating a sample of water from the body of water in the analysis container, decreasing the pressure in the analysis container, measuring the capacitance over the first and second poles, and using the capacitance to determine the volume of gas in the gas collecting space. The device evacuation pump may then be used to empty the gas collecting space 8 and the analysis container 4, so that a new sample may be analysed.

## Claims

1.  A system (1) comprising a heating system or a cooling system and a device for measuring total gas content in a liquid contained in the heating or cooling system, where the heating system or cooling system is a closed system, the device comprising an analysis container (4),

    means for isolating a portion of the liquid (5a, 5b) from the heating or cooling system in the analysis container, means for decreasing the pressure (6) in the analysis container, a gas collecting space (8) in fluid connection with the analysis container and arranged above the analysis container such that gas will be collected in the gas collecting space such that an interface (12) between the liquid and the gas may form in the gas collecting space,

a first electrical pole (9) being elongated and arranged in the gas collecting space such that it extends through the interface between the liquid and the gas, and

a second pole (10) formed by the inner surface of the gas collecting space,

means for determining the capacitance (11) between the first pole and the second pole, such that the determined capacitance is related to the volume of gas collected in the gas collecting space where the gas collecting space is elongated and has a main axis that is non-horizontal and where the gas collecting space has the same width along its main axis and where the first pole is parallel to the main axis of the gas collecting space.

2. The system according to claim 1 where the gas collecting space is vertically arranged on the analysis container.

3. The system according to claim 1 or 2 where the upper part of the analysis container is funnel shaped.

4. The system according to any one of claims 1 to 3 where the device is arranged to achieve a final pressure in the analysis container of 0.5-0.6 bar ATA.

5. A method for determining total gas in a system (1) according to any one of claims 1 to 4, the method comprising the steps of

a) isolating a portion of the liquid in the analysis container (100),
b) decreasing the pressure in the analysis container (101),
c) measuring the capacitance over the first and second poles (102),
d) using the capacitance to determine the volume of gas in the gas collecting space (103).

6. The method of claim 5 where the final pressure in the analysis container is 0.5-0.6 bar ATA.


**Patentansprüche**

1. System (1) mit einem Heizsystem oder einem Kühlsystem und einer Vorrichtung zur Messung des Gesamtgasgehalts in einer in dem Heiz- oder Kühlsystem enthaltenen Flüssigkeit, wobei das Heizsystem oder das Kühlsystem ein geschlossenes System ist, wobei die Vorrichtung umfasst

einen Analysebehälter (4),
Mittel zum Isolieren eines Teils der Flüssigkeit (5a, 5b) von dem Heiz- oder Kühlsystem in dem Analysebehälter ,
Mittel zum Absenken des Drucks (6) im Analysebehälter,
einen Gassammelraum (8), der in Fluidverbindung mit dem Analysebehälter steht und oberhalb des Analysebehälters so angeordnet ist, dass sich in dem Gassammelraum Gas sammelt, sodass sich in dem Gassammelraum eine Schnittstelle (12) zwischen der Flüssigkeit und dem Gas bilden kann,
einen ersten elektrischen Pol (9), der länglich ist und in dem Gassammelraum so angeordnet ist, dass er sich durch die Schnittstelle zwischen der Flüssigkeit und dem Gas erstreckt, und
einen zweiten Pol (10), der von der Innenfläche des Gassammelraums gebildet wird,
Mittel zum Bestimmen der Kapazität (11) zwischen dem ersten Pol und dem zweiten Pol, sodass die ermittelte Kapazität mit dem in dem Gassammelraum gesammelten Gasvolumen in Beziehung steht, wobei der Gassammelraum länglich ist und eine Hauptachse hat, die nicht horizontal ist, und wobei der Gassammelraum die gleiche Breite entlang seiner Hauptachse hat und wobei der erste Pol parallel zur Hauptachse des Gassammelraums ist.

2. System nach Anspruch 1, wobei der Gassammelraum senkrecht auf dem Analysebehälter angeordnet ist.

3. System nach Anspruch 1 oder 2, wobei der obere Teil des Analysebehälters trichterförmig ist.

4. System nach einem der Ansprüche 1 bis 3, wobei die Vorrichtung so angeordnet ist, dass im Analysebehälter ein Enddruck von 0,5-0,6 bar ATA erreicht wird.

5. Verfahren zur Bestimmung des Gesamtgases in einem System (1) nach einem der Ansprüche 1 bis 4, wobei das Verfahren die folgenden Schritte umfasst:

a) Isolieren eines Teils der Flüssigkeit in dem Analysebehälter (100),

b) Absenken des Drucks im Analysebehälter (101)
c) Messen der Kapazität über den ersten und zweiten Pol (102),
d) Verwendung der Kapazität zur Bestimmung des Gasvolumens im Gassammelraum (103).

**6.** Verfahren nach Anspruch 5, wobei der Enddruck im Analysebehälter 0,5-0,6 bar ATA beträgt.


**Revendications**

**1.** Système (1) comprenant un système de chauffage ou un système de refroidissement et un dispositif de mesure de la teneur totale en gaz d'un liquide contenu dans le système de chauffage ou de refroidissement, où le système de chauffage ou de refroidissement est un système fermé, le dispositif comprenant

un récipient d'analyse (4),
des moyens pour isoler une partie du liquide (5a, 5b) du système de chauffage ou de refroidissement dans le récipient d'analyse,
des moyens pour diminuer la pression (6) dans le récipient d'analyse,
un espace de collecte de gaz (8) en connexion de fluide avec le récipient d'analyse et situé au-dessus du récipient d'analyse, de sorte que le gaz soit collecté dans l'espace de collecte de gaz et qu'une interface (12) entre le liquide et le gaz puisse se former dans l'espace de collecte de gaz,
un premier pôle électrique (9) allongé et disposé dans l'espace de collecte des gaz de manière à traverser l'interface entre le liquide et le gaz, et
un second pôle (10) formé par la surface intérieure de l'espace de collecte des gaz,
des moyens pour déterminer la capacité (11) entre le premier pôle et le second pôle, de telle sorte que la capacité déterminée soit liée au volume de gaz collecté dans l'espace de collecte de gaz, où l'espace de collecte de gaz est allongé et a un axe principal qui n'est pas horizontal et où l'espace de collecte de gaz a la même largeur le long de son axe principal et où le premier pôle est parallèle à l'axe principal de l'espace de collecte de gaz.

**2.** Le système selon la revendication 1, dans lequel l'espace de collecte des gaz est disposé verticalement sur le récipient d'analyse.

**3.** Le système selon la revendication 1 ou 2 dans lequel la partie supérieure du récipient d'analyse est en forme d'entonnoir.

**4.** Le système selon l'une des revendications 1 à 3 dans lequel le dispositif est conçu pour atteindre une pression finale dans le récipient d'analyse de 0,5 à 0,6 bar ATA.

**5.** Méthode de détermination du gaz total dans un système (1) selon l'une quelconque des revendications 1 à 4, la méthode comprenant les étapes suivantes

a) isoler une partie du liquide dans le récipient d'analyse (100),
b) diminuer la pression dans le récipient d'analyse (101)
c) mesurer la capacité sur les premier et second pôles (102),
d) utiliser la capacité pour déterminer le volume de gaz dans l'espace de collecte des gaz (103).

**6.** Méthode de la revendication 5 dans laquelle la pression finale dans le récipient d'analyse est de 0,5 à 0,6 bar ATA.

Fig. 1

Fig. 2

Fig. 3

Fig. 4

Fig. 5

100 ISOLATE LIQUID

101 DECREASE PRESSURE

102 MEASURE CAPACITANCE

103 DETERMINE GAS CONTENT

Fig. 6

Fig. 7

**EP 4 511 601 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20150160147 A1
- JP S5282358 A
- US 6272906 B1
- US 6318332 B1